(19) Europäisches Patentamt
European Patent Office
Office européen des brevets

(11) **EP 1 285 906 A2**

(12) **EUROPÄISCHE PATENTANMELDUNG**


(43) Veröffentlichungstag: **26.02.2003 Patentblatt 2003/09**

(21) Anmeldenummer: **02017674.9**

(22) Anmeldetag: **07.08.2002**

(51) Int Cl.[7]: **C07C 69/28**, C07C 69/16, A61K 7/46, A61K 7/50, C11D 3/50

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **20.08.2001 DE 10140787**



(71) Anmelder: **HAARMANN & REIMER GMBH**
**37603 Holzminden (DE)**

(72) Erfinder:
- **Eh, Marcus, Dr.**
  **37603 Holzminden (DE)**
- **Surburg, Horst, Dr.**
  **37603 Holzminden (DE)**
- **Bertram, Heinz-Jürgen, Dr.**
  **37603 Holzminden (DE)**
- **Sonnenberg, Steffen, Dr.**
  **37603 Holzminden (DE)**

(74) Vertreter: **Zobel, Manfred, Dr. et al**
**Bayer AG**
**Bayer Chemicals**
**Legal & Patents**
**Patents and Licensing**
**51368 Leverkusen (DE)**


(54) **Depotpräparate zur gezielten Freisetzung eines Aldehydes oder Ketones, eines Alkohols und einer Carbonsäure**


(57) Die Erfindung betrifft Depotpräparate, die mindestens eine Verbindung der Formel I enthalten, zur gezielten Freisetzung von einem Aldehyd oder Keton zusammen mit einem Alkohol und einer Carbonsäure, wobei es sich bei den freigesetzten Aldehyden oder Ketonen um organoleptische Substanzen, im speziellen um Riechstoffe oder Geschmackstoffe handelt.

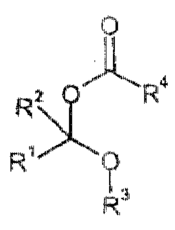

(I)


EP 1 285 906 A2

## Beschreibung

Die Erfindung betrifft Depotpräparate ("Delivery Systems") zur gezielten Freisetzung von einem Aldehyd oder Keton, Alkohol und einer Carbonsäure. Bei den freigesetzten Aldehyden, Ketonen und Alkoholen handelt es sich um organoleptische Substanzen, im speziellen um Riechstoffe oder Geschmackstoffe. Die Depotpräparate sind erhältlich durch Umwandlung von Acetalen in die entsprechenden Enolether und anschließender Umsetzung mit einer Carbonsäure, oder durch Hydrid Reduktion eines Carbonsäureesters bzw. Umsetzung eines Carbonsäureesters mit einem metallorganischen Reagenz und anschließendem Abfang des sich bildenden Halbacetals mit einem Carbonsäureanhydrid.

Die prinzipielle Vorgehensweise zur Parfümierung von Konsumartikeln ist die, dass das Riechstoffe enthaltene Parfümöl direkt mit dem Produkt vermischt wird. Die Probleme die hierbei auftreten sind, dass leichtflüchtige Substanzen während der Einarbeitung in das Produkt oder im Verlauf der Lagerung teilweise oder vollständig durch Ausdampfen verloren gehen. Weiterhin sind zahlreiche Substanzen und hierbei besonders auch Aldehyde instabil unter den gegebenen Bedingungen, was zur teilweisen oder vollständigen Zersetzung dieser Moleküle führt. Die Konsequenz hieraus ist, dass alle Substanzen, die den oben beschriebenen Problemen unterliegen im Endprodukt sensorisch nur noch schwach oder gar nicht mehr wahrnehmbar sind. Dies kann in Einzelfällen zu einer Veränderung des Gesamtgeruchseindrucks der Komposition führen.

Aus WO 94/06441 sind Acetale, Ketale und spezielle Orthoester als im basischen stabile Depotpräparate bekannt. Im sauren Milieu erfolgt, wie z.B. im Kontakt mit der Haut, die Hydrolyse, so dass Alkohole oder Ketone freigesetzt werden.

Ein Depotpräparat zur Freisetzung von Aldehyden, im speziellen Citral, in Lebensmitteln und hier im speziellen in alkoholischen oder nicht-alkoholischen Getränken wird in WO 00/04009 beschrieben. Hierbei handelt es sich um Dicarboalkoxydioxolane, welche durch Acetalisierung aus einem Aldehyd und einem Weinsäurederivat zugänglich sind. Sie besitzen in wässrig sauren alkoholischen und nicht-alkoholischen Getränken eine sehr viel höhere Halbwertszeit als vergleichbare Acetale.

WO 00/38616 beansprucht ein zyklisches Dioxaketon, welches nach Hydrolyse gleichzeitig ein Aldehyd oder Keton und eine Hydroxycarbonsäure freisetzt. Die Herstellung dieser Verbindungen gelingt durch Umsetzung der entsprechenden Hydroxycarbonsäure mit dem Aldehyd oder Keton unter Zugabe katalytischer Mengen Säure am Wasserabscheider. Bevorzugt sind Aldehyde oder Ketone mit Riechstoffeigenschaften und $\alpha$-Hydroxycarbonsäuren oder ringsubstituierte Benzoesäuren.

Keines der oben beschriebenen Depotpräparate besitzt jedoch die Fähigkeit simultan drei Komponenten mit drei unterschiedlichen funktionellen Gruppen freizusetzen. Weiterhin werden die Probleme, die bei der konventionellen Parfümierung von Konsumartikeln auftreten (Ausdampfen während der Lagerung, Instabilität usw.), durch das erfindungsgemäße Depotpräparat gelöst.

Gegenstand der Erfindung sind Depotpräparate enthaltend mindestens eine Verbindung der Formel (I)

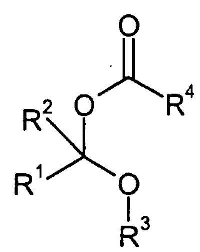

(I),

worin

$R^1$, $R^3$ und $R^4$ — unabhängig voneinander — einen organischen Rest mit 1 bis 30 Kohlenstoffatomen bedeuten,

$R^2$ Wasserstoff oder einen organischen Rest mit 1 bis 30 Kohlenstoffatomen bedeutet und die Verbindung der Formel (I) nach Hydrolyse oder enzymatischer Spaltung neben dem Aldehyd oder Keton noch einen Alkohol und eine Carbonsäure freisetzt.

In einer bevorzugten Ausführungsform der Erfindung werden Verbindungen der Formel (I) eingesetzt, in denen

$R^1$ und $R^3$ unabhängig voneinander für einen gesättigten oder ungesättigten, linearen oder verzweigten, alicyclischen oder aromatischen Rest mit 1 bis 18 Kohlenstoffatomen stehen, der gegebenenfalls noch Hete-

roatome enthalten kann,

$R^2$ für Wasserstoff, einen gesättigten oder ungesättigten, linearen oder verzweigten, alicyclischen oder aromatischen Rest mit 1 bis 18 Kohlenstoffatomen steht, der gegebenenfalls noch Heteroatome enthalten kann,

$R^4$ für einen gesättigten oder ungesättigten, linearen oder verzweigten, alicyclischen oder aromatischen Rest mit 1 bis 22 Kohlenstoffatomen steht, der gegebenenfalls noch Heteroatome enthalten kann und der zudem gegebenenfalls mit ionischen Substituenten substituiert sein kann.

Bevorzugte Heteroatome sind Sauerstoff und Schwefel und besonders bevorzugt ist Sauerstoff.

Beispiele für ionische Substituenten sind $-CO_2M$ und $—OCO_2M$, wobei M ein Alkalimetall ist.

Die erfindungsgemäßen 1-Alkoxyester der Formel (I) zerfallen einerseits nach Hydrolyse in wässrigem Medium, bevorzugt in sauer wässrigem Medium mit einem pH < 6, wie auch in alkalisch wässrigem Medium mit einem pH > 9, und andererseits nach enzymatischer Spaltung, so dass ein Aldehyd oder Keton, sowie ein Alkohol und eine Carbonsäure freigesetzt werden, die dann ihre Wirkung (z.B. Duftfreisetzung) entfalten und sich an Substrate anheften können.

Überraschenderweise lässt sich die Freisetzungsrate über die Reste $R^3$ und $R^4$, in Abhängigkeit von $R^1$ und $R^2$, in der Art steuern, dass 1-Alkoxyester mit kleinen und schmalen Resten $R^3$ und $R^4$ kurze Halbwertszeiten besitzen, wohingegen voluminöse und langkettige Reste $R^3$ und $R^4$ die Halbwertszeit erhöhen. Dies ist insofern von Bedeutung, da somit 1-Alkoxyester der Formel (I) mit unterschiedlichen Freisetzungsprofilen hergestellt werden können. Darauf aufbauend können das Depotpräparat und die Formulierung in ihren jeweiligen Resten bzw. Inhaltsstoffen aufeinander abgestimmt werden, um durch kontrollierte Freisetzung bzw. kontrollierte Haftung/Übertragung eine applikationsorientierte Freisetzung zu erzielen.

Die enzymatische Spaltung kann bevorzugt durch Esterasen oder Lipasen durchgeführt werden.

In einer weiteren bevorzugten Ausführungsform der Erfindung hat der aus der Verbindung der Formel (I) freigesetzte Aldehyd $R^1COH$ oder das Keton $R^1R^2CO$ ein Molekulargewicht von 100 g/mol bis 350 g/mol und besonders bevorzugt von 120 g/mol bis 270 g/mol. Weiterhin ist bevorzugt, wenn der freigesetzte Aldehyd oder das Keton ein Riechstoff oder Geschmackstoff ist.

Nicht limitierende Beispiele für Aldehyde, die nach Spaltung des erfindungsgemäßen Depotpräparates freigesetzt werden können, sind im folgenden genannt:

Phenylacetaldehyd, p-Methylphenylacetaldehyd, p-Isopropylphenylacetaldehyd, Methylnonyl acetaldehyd, phenylpropanal, 3-(4-t-Butylphenyl)-2-methylpropanal (Lilial), 3-(4-t-Butylphenyl)-propanal (Bourgeonal), 3-(4-Methoxyphenyl)-2-methylpropanal (Canthoxal), 3-(4- Isopropylphenyl)-2-methylpropanal (Cymal), 3-(3,4-Methylendioxyphenyl)-2-methylpropanal (Helional), 3-(4-Ethylphenyl)-2,2-dimethylpropanal (Floralozone), Phenylbutanal, 3-Methyl-5-phenylpentanal, Hexanal, trans-2-Hexenal, cis-Hex-3-enal, Heptanal, cis-4-Heptenal, 2-Ethyl-2-heptenal, 2,6-Dimethyl-5-heptenal (Melonal), 2,4-Heptadienal, Octanal, 2-Octenal, 3,7-Dimethyloctanal, 3,7-Dimethyl-2,6-octadien-1-al, 3,7-Dimethyl-2,6-octadien-3-al, 3,7-Dimethyl-6-octenal (Citronellal), 3,7-Dimethyl-7-hydroxyoctan-1-al (Hydroxy Citronellal), Nonanal, 6-Nonenal, 2,4-Nonadienal, 2,6-Nonadienal, Decanal, 2-Methyldecanal, 4-Decenal, 9-Decenal, 2,4-Decadienal, Undecanal, 2-Methyldecanal, 2-Methylundecanal, 2,6,10-Trimethyl-9-undecenal (Adoxal), Undec-10-enylaldehyd, Undec-8-enanal, Dodecanal, Tridecanal, Tetradecanal, Anisaldehyd, Zimtaldehyd, α-Amylzimtaldehyd, α-Hexylzimtaldehyd, Methoxyzimtaldehyd, Isocyclocitral, Citronellyloxyacetaldehyd, Cortexaldehyd, Cuminaldehyd, Cyclamenaldehyd, Florhydral, Heliotropin, Hydratropaldehyd, Vanillin, Ethylvanillin, Benzaldehyd, p-Methylbenzaldehyd, 3,4-Dimethoxybenzaldehyd, 3- und 4-(4-Hydroxy-4-methylpentyl)-3-cyclohexen-1-carboxaldehyd (Lyral), 2,4-Dimethyl-3-cyclohexen-1-carboxaldehyd (Triplal), I-Methyl-3-(4-methylpentyl)-3-cyclohexencarboxaldehyd (Vernaldehyd) oder p-Methylphenoxyacetaldehyd (Xi Aldehyd) ist.

Nicht limitierende Beispiele für Ketone, die nach Spaltung des erfindungsgemäßen Depotpräparates freigesetzt werden können, sind im folgenden genannt:

α-Damascon, β-Damascon, δ-Damascon, β-Damascenon, Muscon, 6,7-Dihydro-1,1,2,3,3-pentamethyl-4 (5H)-indanon (Cashmeran), *cis*-Jasmon, Dihydrojasmon, α-Ionon, β-Ionön, Dihydro-β-ionon, γ-Methylionon, α-*iso*-Methylionon, 4-(3,4-methylendioxyphenyl)butan-2-on, 4-(4-Hydroxyphenyl)butan-2-on, Methyl-β-naphthylketon, Methylcedrylketon, 6-Acetyl-1,1,2,4,4,7-hexamethyltetralin (Tonalid), *l* Carvon, 5-Cyclohexadecen-1-on, Acetophenon, Decaton, p-Hydroxyphenylbutan-2-on, 2-[2-(4-Methyl-3-cyclohexenyl-1-yl)propyl]cyclopentan-2-on, 2-*sec*-Butylcyclohexanon, β-Dihydroionon, Allylionon, α-Iron, α-Ceton, α-Irison, Acetanisole, Geranylaceton, 1-(2-Methyl-5-isopropyl-2-cyclohexenyl)-1-propanon, Acetyldiisoamylen, Methylcyclocitron, 4-t-Pentylcyclohexanone, p-*t*-Butylcyclohexanon, *o-t*-Butylcyclohexanon, Ethylamylketon, Ethylpentylketon, Menthon, Methyl-7,3-Dihydro-2H-1,5-benzodioxepin-3-on, Fenchon.

Nicht limitierende Beispiele für Alkohole, die nach Spaltung des erfindungsgemäßen Depotpräparates freigesetzt werden können, sind im folgenden genannt:

Methanol, 2,4-Dimethyl-3-cyclohexen-1-methanol (Floralol), 2,4-Dimethyl cyclohexanmethanol (Dihydrofloralol), 5,6-Dimethyl-1-methylethenylbicyclo[2.2.1]hept-5-en-2-methanol (Arbozol), 2,4,6-Trimethyl-3 -cyclohexen-1-methanol (Isocyclogeraniol), 4-(1-Methylethyl)cyclo-hexanmethanol (Mayol), α-3,3-Trimethyl-2-norboranmethanol, 1,1-Dimethyl-1-(4-methylcyclohex- 3-enyl)methanol, Ethanol, 2-Phenylethanol, 2-Cyclohexylethanol, 2-(o-Methylphenyl)-ethanol, 2-(m-Methylphenyl)ethanol, 2-(p-Methylphenyl)ethanol, 6,6-Dimethylbicyclo-[3.1.1]hept-2-en-2-ethanol (Nopol), 2-(4-Methylphenoxy)ethanol, 3,3-Dimethyl-$\Delta^2$-β-norbomanethanol, 2-Methyl-2-cyclohexylethanol, 1-(4-Isopropylcyclohexyl)-ethanol, 1-Phenylethanol, 1,1-Dimethyl-2-phenylethanol, 1,1-Dimethyl-2-(4-methyl-phenyl)-ethanol, n-Propanol, 2-Propanol, 1-Phenylpropanol, 3-Phenylpropanol, 2-Phenylpropanol (Hydrotropic Alkohol), 2-(Cyclododecyl)propan-1-ol (Hydroxyambran), 2,2-Dimethyl-3-(3-methylphenyl)propan-1-ol (Majantol), 2-Methyl-3-phenylpropanol, 3-Phenyl-2-propen-1-ol (Zimtalkohol), 2-Methyl-3-phenyl-2-propen-1-ol (Methylzimtalkohol), α-n-Pentyl-3-phenyl-2-propen-1-ol (α-Amylzimtalkohol), 3-Hydroxy-3-phenylpropionsäureethylester, 2-(4-Methylphenyl)-2-propanol, n-Butanol, 2-Butanol, 3-Methylbutanol, 3-(4-Methylcyclohex-3-en)-butanol, 2-Methyl-4-(2,2,3-trimethyl-3-cyclopenten-1-yl)butanol, 2-Ethyl-4-(2,2,3 -trimethyl-cyclopent-3-enyl)-2-buten-1-ol, 3-Methyl-2-buten-1-ol, 2-Methyl-4-(2,2,3-trimethyl-3-cyclopenten-1-yl)-2-buten-1-ol, 3-Hydroxy-2-butanon, 3-Hydroxybuttersäureethylester, 4-Phenyl-3-buten-2-ol, 2-Methyl-4-phenylbutan-2-ol, 4-(4-Hydroxyphenyl)butan-2-on, 4-(4-Hydroxy-3-methoxyphenyl)butan-2-on, Pentanol, *cis*-3-Pentenol, 3-Methylpentanol, 3-Methyl-3-penten-1-ol, 2-Methyl-4-phenylpentanol (Pamplefleur), 3-Methyl-5-phenylpentanol (Phenoxanol), 2-Methyl-5-phenylpentanol, 2-Methyl-5-(2,3-dimethyltricyclo[2.2.1.0(2,6)]hept-3-yl)-2-penten-1-ol (Santalol), 4-Methyl-1-phenyl-2-pentanol, (1-Methyl-bicyclo[2.1.1]hepten-2-yl)-2-methylpent-1-en-3-ol, 3-Methyl-1-phenylpentan-3-ol, 1,2-Dimethyl-3-(1-methylethenyl)cyclopentan-1-ol, 2-Isopropyl-5-methyl-2-hexenol, *cis*-3-Hexen-1-ol, *trans*-2-Hexen-1-ol, 2-Isoproenyl-4-methyl-4-hexen-1-ol (Lavandulol), 2-Ethyl-2-prenyl-3-hexenol, 1-Hydroxymethyl-4-iso-propenyl-1-cyclohexen (Dihydrocumin Alkohol), 1-Methyl-4-isopropenylcyclohex-6-en-2-ol (Carvenol), 6-Methyl-3-isopropenylcyclohexan-1-ol, 1-Methyl-4-iso-propenylcyclohexan-3-ol, 4-Isopropyl-1-methylcyclohexan-3-ol, 4-tert-Butylcyclohexanol, 2-tert-Butylcyclohexanol, 2-tert-Butyl-4-methylcyclohexanol, 4-Isopropylcyclohexanol, 4-Methyl-1-(1-methylethyl)-3-cyclohexen-1-ol, 2-(5,6,6-trimethyl-2-norbornyl)cyclohexanol, Isobornylcyclohexanol, 3,3,5-Trimethylcyclohexanol, 1-Methyl-4-isopropylcyclohexan-3-ol, 1,2-Dimethyl-3-(1-methylethyl)-cyclohexan-1-ol, Heptanol, 2,4-Dimethylheptan-1-ol, 2,4-Dimethyl-2,6-heptandienol, 6,6-Dimethyl-2-oxymethylbicyclo [3.1.1]hept-2-en (Myrtenol), 4-Methyl-2,4-heptadien-1-ol, 3,4,5,6,6-Pentamethyl-2-heptanol, 3,6-Dimethyl-3-vinyl-5-hepten-2-ol, 6,6-Dimethyl-3-hydroxy-2-methylenbicyclo[3.1.1 ]heptane, 1,7,7-Trimethylbicyclo[2.2.1]heptan-2-ol, 2,6-Dimethylheptan-2-ol, 2,6,6-Trimethylbicyclo[1.3.3]heptan-2-ol, Octanol, 2-Octenol, 2-Methyloctan-2-ol, 2-Methyl-6-methylen-7-octen-2-ol (Myrcenol), 7-Methyloctan-1-ol, 3,7-Dimethyl-6-octenol, 3,7-Dimethyl-7-octenol, 3,7-Dimethyl-6-octen-1-ol (Citronellol), 3,7-Dimethyl-2,6-octadien-1-ol (Geraniol), 3,7-Dimethyl-2,6-octadien-1-ol (Nerol), 3,7-Dimethyl-1,6-octadien-3-ol (Linalool), 3,7-Dimethyloctan-1-ol (Pelagrol), 3,7-Dimethyloctan-3-ol (Tetrahydrolinalool), 2,4-Octadien-1-ol, 3,7-Dimethyl-6-octen-3-ol, 2,6-Dimethyl-7-octen-2-ol, 2,6-Dimethyl-5,7-octadien-2-ol, 4,7-Dimethyl-4-vinyl-6-octen-3-ol, 3-Methyloctan-3-ol, 2,6-Dimethyloctan-2-ol, 2,6-Dimethyloctan-3-ol, 3,6-Dimethyloctan-3-ol, 2,6-Dimethyl-7-octen-2-ol, 2,6-Dimethyl-3,5-octadien-2-ol (Muguol), 3-Methyl-1-octen-3-ol, 7-Hydroxy-3,7-dimethyloctanal, 3-Nonanol, 2,6-Nonadien-1-ol, *cis*-6-Nonen-1-ol, 6,8-Dimethylnonan-2-ol, 3-(Hydroxymethyl)-2-nonanon, 2-Nonen-1-ol, 2,4-Nonadien-1-ol, 3,7-Dimethyl-1,6-nonadien-3-ol, Decanol, 9-Decenol, 2-Decen-1-ol, 2,4-Decadien-1-ol, 4-Methyl-3-decen-5-ol, 3,7,9-Trimethyl-1,6-decadien-3-ol (Isobutyl Linalool), Undecanol, 2-Undecen-1-ol, 10-Undecen-1-ol, 2-Dodecen-1-ol, 2,4-Dodecadien-1-ol, 2,7,11-Trimethyl-2,6,10-dodecatrien-1-ol (Farnesol), 3,7,11-Trimethyl-1,6,10-dodecatrien-3-ol, 3,7,11,15-Tetramethylhexadec-2-en-1-ol (Phytol), 3,7,11,15-Tetramethylhexadecll-en-3-ol (iso Phytol), Benzylalkohol, p-Methoxybenzylalcohol (Anisalkohol), *para*-Cymen-7-ol (Cuminalkohol), 4-Methylbenzylalkohol, 3,4-Methylendioxybenzylalkohol, Methylsalicylat, Benzylsalicylat, cis-3-Hexenylsalicylat, n-Pentylsalicylat, 2-Phenylethylsalicylat, n-Hexylsalicylat, 2-Methyl-5-isopropylphenol, 4-Ethyl-2-methoxyphenol, 4-Allyl-2-methoxyphenol (Eugenol), 2-Methoxy-4-(1-propenyl)phenol (Isoeugenol), 4-Allyl-2,6-dimethoxy-phenol, 4-tert-Butylphenol, 2-Ethoxy-4-methylphenol, 2-Methyl-4-vinylphenol, 2-Isopropyl-5-methylphenol (Thymol), *ortho*-Hydroxybenzoesäurepentylester, 2-Hydroxybenzoesäureethylester, 2,4-Dihydroxy-3,6-dimethylbenzoesäuremethylester, 3-Hydroxy-5-methoxy-1-methylbenzol, 2-tert-Butyl-4-methyl-1-hydroxybenzol, 1-Ethoxy-2-hydroxy-4-propenylbenzol, 4-Hydroxytoluol, 4-Hydroxy-3-methoxybenzaldehyd, 2-Ethoxy-4-hydroxybenzaldehyd, Decahydro-2-naphthol, 2,5,5-Trimethyl-octahydro-2-naphthol, 1,3,3-Trimethyl-2-norbomanol (Fenchol), 3a,4,5,6,7,7a-Hexahydro-2,4-dimethyl-4,7-methano-1H-inden-5-ol, 3a,4,5,6,7,7a-Hexahydro-3,4-dimethyl-4,7-methano-1H-inden-5-ol, 2-Methyl-2-vinyl-5-(1-hydroxy-1-methylethyl)tetrahydrofuran.

Nicht limitierende Beispiele für Carbonsäuren, die nach Spaltung des erfindungsgemäßen Depotpräparates freigesetzt werden können, sind im folgenden genannt:

unsubstituierte, gesättigte Monocarbonsäuren (z.B. Essigsäure, Propionsäure; Buttersäure, Valeriansäure, Capronsäure, Caprylsäure, Pelargonsäure, Caprinsäure, Laurinsäure, Myristinsäure, Palmitinsäure, Stearinsäure); dialkylsubstituierte Essigsäuren (z.B. 2-Butyloctansäure, 2-Butyldecansäure, 2-Hexyloctansäure, 2-Hexyldecansäure); einfach oder mehrfach ungesättigte Monocarbonsäuren (z.B. Ölsäure, Linolsäure, α-Linolensäure, γ-Linolensäure);

Alkin-, Alkadiin-, oder Alkatriincarbonsäuren (z.B. Non-8-insäure, Dec-9-insäure, Tridec-9-insäure, 13-Methyltetradec-9-insäure, Pentadec-6-insäure, Pentadec-7-insäure, Hexadec-9-insäure, 15-Methylhexadec-9-insäure, Heptadec-2-insäure, Heptadec-9-insäure, Octadec-12-insäure, Octadec-6,12-diinsäure, Nonadec-9-insäure); α-Hydroxycarbonsäuren (z.B. α-Hydroxyvaleriansäure, α-Hydroxycapronsäure, α-Hydroxycaprylsäure, α-Hydroxypelargonsäure, α-Hydroxycaprinsäure, α-Hydroxylaurinsäure, α-Hydroxymyristinsäure, α-Hydroxypalmitinsäure, α-Hydroxypalmitinsäure); unsubstituierte α,ω-Alkandicarbonsäuren (z.B. Malonsäure, Bernsteinsäure, Glutarsäure, Adipinsäure, Pimelinsäure, Korksäure, Azelainsäure).

Eine weitere bevorzugte Ausführungsform des erfindungsgemäßen Depotpräparates beinhaltet 1-Alkoxyester der Formel (I), welche eine geringe Löslichkeit in Wasser besitzen und somit eine relativ große Tendenz haben sich an Substrate anzuheften, bzw. sich im Gasraum über einer wässrigen Lösung anzureichern. Wünschenswert ist hierbei, dass die erfindungsgemäßen 1-Alkoxyester der Formel (I) eine geringere Wasserlöslichkeit als die freiwerdende Carbonsäure haben, so dass eine bessere Anheftung auch der Carbonsäure an das Substrat erfolgen kann, bzw. eine Anreicherung im Gasraum über der wässrigen Phase stattfindet. Löslichkeiten können direkt gemessen oder einfacher durch Heranziehung von Octanol/Wasser Verteilungskoeffizienten (log P Wert) bestimmt werden. Der log P Wert ist in der Literatur (A. Leo, C. Hansch und D. Elkins, Chem. Rev., **71**, 1971, 525-616; C. Hansch, J.E. Quinlan, G.L. Lawrence, J. Org. Chem., **33**, 1968, 347-350) eine etablierte Größe zur Bestimmung der Lipophilie.

Für Moleküle mit einem niedrigen log P Wert ist der Übergang aus einem wässrigen System an ein Substrat sehr schwierig, vielmehr haben solche Moleküle die Tendenz sich zu lösen und weggespült zu werden. Dies trifft insbesondere auf saure Verbindungen zu. Die vorliegende Erfindung kann diese Problematik beheben, da der Übergang der Carbonsäure mit einem niedrigen log P Wert aus der wässrigen Lösung an ein Substrat in der chemisch gebundenen Form der erfindungsgemäßen 1-Alkoxyester der Formel (I) mit einem höheren log P Wert erfolgt. Das bedeutet, dass die Übertragung einer Carbonsäure nach Freisetzung aus dem Depotpräparat mit einer höheren Rate erfolgt als die Übertragung der freien Carbonsäure. Die Freisetzung erfolgt nach anschließender Spaltung des erfindungsgemäßen Depotpräparates.

Die kontrollierte Freisetzung des Aldehyds oder Ketons, des Alkohols und der Carbonsäure aus dem erfindungsgemäßen Depotpräparat kann zur Behandlung (z.B. Beduftung) von einer Fülle an Substraten, wie z.B. Haare, menschliche Haut, Wäsche und harte Oberflächen, benutzt werden.

Beispiele für Riechstoffe, mit denen sich das erfindungsgemäße Depotpräparat vorteilhaft kombinieren lässt, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3rd. Ed., Wiley-VCH, Weinheim 1997.

Im einzelnen seien genannt:

Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;

Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;

der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;

der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;

der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;

der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinoat; Methyl-2-noninoat; Allyl-2-isoamyloxyacetat; Methyl-3,7-dimethyl-2,6-octadienoat;

der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;

der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;

der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;

der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7, 8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);

der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;

der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;

der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid;

2-(2,4-Dimethyl-3-cyclohexen-1-yl)-5-methyl-5-(1-methylpropyl)-1,3-dioxan;

der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;

der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;

der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;

der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;

der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;

der aromatischen Kohlenwasserstoffe wie z. B. Styrol und Diphenylmethan;

der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl) propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;

der Ester von araliphatischen Alkoholen mit aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Plienylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno [1,2-d]-m-dioxin; 4,4a,5,9b-Tetrahydro-2,4-dimethylindeno[1,2-d]-m-dioxin; der aromatischen oder araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;

der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;

der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;

der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3 -methoxypyrazin;

der Phenole, Phenylether oder Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;

der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;

der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cisund trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Riechstoffe oder Parfümöle, die das erfindungsgemäße Depotpräparat enthalten, können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können Riechstoffe oder Parfümöle, die das erfindungsgemäße Depotpräparat enthalten, an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Riechstoffe oder Parfümöle, die das erfindungsgemäße Depotpräparat enthalten, können auch mikroverkapselt, sprühgetrocknet, als Einschluss-komplex oder als Extrusions-Produkt vorliegen und in dieser Form dem zu parfümierenden Produkt hinzugefügt werden.

Gegebenenfalls können die Eigenschaften der derart modifizierten Riechstoffe oder Parfümöle durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Riechstoffe oder Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, gegebenenfalls in einem geeigneten Lösungsmittel, z.B. Isopropanol, erfolgen.

In Parfümkompositionen beträgt die eingesetzte Menge des erfindungsgemäßen Depotpräparates 0,01 bis 75 Gew.%, vorzugsweise 0,05 bis 50 Gew.-%, besonders bevorzugt ist eine Einsatzmenge von 0,5 bis 20 %, bezogen auf das gesamte Parfümöl.

Riechstoffe oder Parfümöle, die das erfindungsgemäße Depotpräparat enthalten, können in konzentrierter Form, in Lösungen oder in oben beschriebener modifizierter Form für die Herstellung von z.B. kosmetischen Pflegeprodukten verwendet werden. Hierbei und insbesondere für Waschprodukte oder andere Produktapplikationen ist ein langanhaltender Geruchseindruck auf der Haut oder dem Haar erwünscht. Als Beispiele seien hier genannt: Parfüm-Extraits, Eau de Parfums, Eau de Toilettes, Rasierwässer, Eau de Colognes, Pre-shave-Produkte, Splash-Colognes,

Körperpflegemittel wie z.B. feste und flüssigen Seife, Duschgele, Shampoos, Rasierseifen, Rasierschäume, Badeöle, kosmetische Emulsionen vom Öl-in-Wasser-, vom Wasserin-Öl- und vom Wasser-in-Öl-in-Wasser-Typ wie z.B. Hautcremes- und — lotionen, Gesichtscremes und — lotionen, Sonnenschutzcremes-und — lotionen, After-sun-cremes und — lotionen, Handcremes und — lotionen, Fußcremes und — lotionen, Enthaarungscremes und — lotionen, After-shave-Cremes und — lotionen, Bräunungscremes und — lotionen, Haarpflegeprodukten wie z.B. Haarsprays, Haargelen, festigende Haarlotionen, Haarspülungen, permanenten und semipermanenten Haarfärbemitteln, Haarverformungsmitteln wie kaltwellen und Haarglättungsmitteln, Haarwässern, Haarcremes und — lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara.

Weiterhin können Riechstoffe oder Parfümöle, die das erfindungsgemäße Depotpräparat enthalten, in konzentrierter Form, in Lösungen oder in oben beschriebener modifizierter Form für die Herstellung von z.B. Haushaltprodukten, wie Fußbodenreinigem, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln eingesetzt werden.

Darüber hinaus kann das erfindungsgemäße Depotpräparat zur Aromatisierung von beispielsweise Verpakkungsprodukten oder Nahrungsmitteln sowie deren Anwendungsformen für die Verwendung als Lebensmittel zum menschlichen oder tierischen Verzehr eingesetzt werden.

Im besonderen bevorzugte zu aromatisierende Produkte sind z.B. Süßwaren, Backwaren, Schokoladen, Gelatinewaren, Bonbons, alkoholische Getränke, nicht alkoholische Getränke, Eiscreme, Joghurt, Milchgetränke, Suppen, Soßen, Snacks, Kaugummi, Mundwasser, Fleisch- und Wurstwaren, Pflanzenproteinwaren, Fisch, Käse und Babynahrung.

Die erfindungsgemäßen 1-Alkoxyester der Formel (I) können nach dem Fachmann wohlvertrauten Methoden hergestellt werden. In einem ersten Syntheseweg setzt man den Aldehyd $R^1CHO$ oder das Keton $R^1R^2CO$ mit 2 bis 4 Äquivalenten Alkohol $R^3OH$ in Gegenwart von katalytischen Mengen Säure zu dem entsprechenden Acetal um (J. A. Barth, Organikum, 20$^{th}$ Edition, J.A. Barth Verlag, Leipzig 1996). Anschließend spaltet man unter saurer Katalyse aus dem Acetal ein Äquivalent Alkohol ab und gelangt so zu dem entsprechenden Enolether. Säuren, die man für die Alkoholabspaltung zu den Enolethern benutzen kann sind z.B. Trimethylsulfonsäuretrimethylsilylester (Gassman, P. G.; Burns, S.J.; Pfister, K.B., J. Org. Chem., **58**, 1449-1457, 1993), Bortrifluorid (Faja, M.; Reese, C.B.; Song, Q.; Zhang, P.-Z., J. Chem. Soc. Perkin Trans. 1, 191-194, 1997), p-Toluolsulfonsäure (Greco, C.V.; Grosso, V.G., J. Org. Chem., **38**, 146-151, 1973), Schwefelsäure (Eliel, E.L; Krishnamurthy, S., J. Org. Chem., **30**, 848-854, 1965), Eisenchlorid (Fadel, A.; Yefsah, R.; Salaun, J., Synthesis, 37-39, 1987) und Phosphorsäure (Ferwanah, A.; Pressler, W.; Reichardt, C., Tetrahedron Lett., **40**, 3979-3982, 1973). Nachfolgende sauer katalysierte Umsetzung des Enolethers mit einer Carbonsäure $R^4CO_2H$ liefert die 1-Alkoxyester (Boons, G.-J.; Downham, R.; Kim, K.S.; Ley, S.V.; Woods, M., Tetrahedron, **50**, 7157-7176, 1994).

In einem zweiten Syntheseweg zur Herstellung der erfindungsgemäßen 1-Alkoxyester der Formel (I) setzt man einen Carbonsäureester $R^1CO_2R^3$ mit einem Aluminiumhydrid Reduktionsmittel, bevorzugt Diisobutylaluminiumhydrid, bei einer Temperatur T = < 0°C, bevorzugt T = < -60°C um, und gibt anschließend ein Carbonsäureanhydrid $(R^4CO)_2O$ hinzu, um das sich bildende Halbacetal abzufangen (Kopecky, D.J.; Rychnovsky, S.D., J. Org. Chem., **65**, 191-198, 2000). Weiterhin kann der Carbonsäureester $R^1CO_2R^3$ mit einem metallorganischen Reagenz $R^2M$ bei einer Temperatur T = < 0°C, bevorzugt T = < -60°C umgesetzt werden und anschließend fängt man das sich bildende Halbacetal mit einem Carbonsäureanhydrid $(R^4CO)_2O$ ab (Hayakawa, H.; Miyazawa, M.; Tanaka, H.; Miyasaka, T., Nucleosides Nucleotides, **13**, 297-308, 1994).

Die folgenden, nicht limitierenden Beispiele erläutern die Erfindung.

**<u>1. Allgemeine Vorschrift zur Herstellung der 1-Alkoxyester</u>**

Man legt 0,65 mol Alkohol und 0,16 mol Aldehyd in 150 ml Toluol vor und fügt 16 mmol Citronensäure hinzu. Jetzt erhitzt man am Wasserabscheider bis sich keine sichtbaren Mengen Wasser mehr abscheiden. Nach beendeter Reaktion destilliert man das Toluol und den überschüssigen Alkohol ab, nimmt den Rückstand in Ether auf und wäscht die organische Phase noch zweimal mit gesättigter $NaHCO_3$-Lösung. Das entstandene Acetal kann ohne weitere Reinigung in die Folgereaktion eingesetzt werden.

100 mmol Acetal und 180 mmol N,N'-Diisopropylamin werden in 150 ml $CH_2Cl_2$ vorgelegt und auf — 20°C abgekühlt. Nun fügt man 165 mmol Trifluormethansulfonsäuretrimethylsilylester so zu, dass die Temperatur — 15°C nicht übersteigt. Nach beendetem Zutropfen lässt man auf Raumtemperatur kommen und rührt bis die Reaktion beendet ist. Anschließend fügt man 200 ml 1M NaOH hinzu, rührt 5 Minuten und trennt die Phasen. Die organische Phase wird noch zweimal mit 1M NaOH gewaschen, nachfolgend getrocknet, abfiltriert und eingeengt. Die Reinigung des

rohen Enolethers erfolgt mittels Chromatographie an Kieselgel.

20 mmol des Enolethers und 25 mmol der Carbonsäure werden in 30 ml Toluol vorgelegt und auf 0°C abgekühlt. Anschließend gibt man 0,02 mmol p-Toluolsulfonsäure hinzu, rührt 2h bei obiger Temperatur und entfernt anschließend die Kühlung. Nach beendeter Reaktion wird die Reaktionslösung mit Diethylether verdünnt und zweimal mit gesättigter $NaHCO_3$-Lösung gewaschen. Nachfolgend wird die organische Phase getrocknet, abfiltriert und eingeengt. Der rohe 1-Alkoxyester wird mittels Chromatographie an Kieselgel gereinigt.

**2. Allgemeine Vorschrift zur Herstellung der 1-Alkoxyester**

Man legt 10 mmol Carbonsäureester in 60 ml Dichlormethan bei —78°C vor und tropft 20 mmol Diisobutylaluminiumhydrid so zu, dass die Temperatur von —65°C nicht überschritten wird. Anschließend gibt man 30 mmol Pyridin, gefolgt von 20 mmol N,N-Dimethylaminopyridin, gelöst in 30 ml Dichlormethan, so zu, dass die Temperatur von —65°C nicht überschritten wird. Im Anschluss gibt man 60 mmol Carbonsäureanhydrid hinzu und rührt weitere 16 Stunden bei —78°C. Jetzt lässt man die Reaktionslösung auf 0°C erwärmen, rührt noch weitere 30 Minuten bei 0°C und gibt bei 0°C 100 ml kalte, gesättigte $NH_4Cl$-Lösung hinzu. Jetzt lässt man auf Raumtemperatur kommen, trennt die Phasen und extrahiert die wässrige Phase noch dreimal mit je 150 ml Dichlormethan. Die vereinigten organischen Phasen werden noch je einmal mit gesättigter $NaHCO_3$-Lösung und gesättigter NaCl-Lösung gewaschen, anschließend mit $Na_2SO_4$ getrocknet, abfiltriert und am Rotationsdampfer von Lösungsmittel befreit. Der rohe 1-Alkoxyester wird mittels Chromatographie an Kieselgel gereinigt.

**Beispiel 1:**

Nonansäure-3-(4-tert-butylphenyl)-1-methoxypropylester

$^1$H-NMR (200 MHz, $CDCl_3$): δ (ppm) = 0.87 (t, J = 6.5 Hz, 3H), 1.20-1.38 (m, 10H), 1.31 (s, 9H), 1.52-1.70 (m, 2H), 1.94-2.07 (m, 2H), 2.28-2.37 (m, 2H), 2.61-2.72 (m, 2H), 3.42 (s, 3H), 5.78 (t, J = 5.4 Hz, 1H), 7.12 (d, J = 8.3 Hz, 2H), 7.31 (d, J = 8.3 Hz, 2H).
$^{13}$C-NMR (50 MHz, $CDCl_3$): δ (ppm) = 14.1, 22.6, 24.9, 29.1, 29.2, 29.3, 29.5, 31.3 (3C), 31.7, 34.3, 34.4, 35.7, 56.5, 98.9, 125.2 (2C), 127.8 (2C), 137.8, 148.6, 173.6.

**Beispiel 2:**

Heptansäure-3-(4-tert-butylphenyl)-1-methoxypropylester

$^1$H-NMR (200 MHz, $CDCl_3$): δ (ppm) = 0.88 (t, J = 6.8 Hz, 3H), 1.21-1.38 (m, 6H), 1.31 (s, 9H), 1.55-1.72 (m, 2H), 1.94-2.07 (m, 2H), 2.28-2.37 (m, 2H), 2.61-2.72 (m, 2H), 3.42 (s, 3H), 5.78 (t, J = 5.3 Hz, 1H), 7.11 (d, J = 8.6 Hz, 2H), 7.33 (d, J = 8.6 Hz, 2H).
$^{13}$C-NMR (50 MHz, $CDCl_3$): δ (ppm) = 14.0, 22.5, 24.9, 28.8, 29.5, 31.3 (3C), 31.4, 34.3, 34.4, 35.8, 56.5, 98.9, 125.2 (2C), 127.8 (2C), 137.8, 148.6, 173.5.

**Beispiel 3:**

Nonansäure-1-[(2-ethylhexyl)oxy]ethylester

$^1$H-NMR (200 MHz, $CDCl_3$): δ (ppm) = 0.82-0.94 (m, 9H), 1.22-1.40 (m, 18H), 1.38 (d, J = 5.2 Hz, 3H), 1.55-1.72 (m, 3H), 2.32 (t, J = 7.5 Hz, 2H), 3.42 (ddd, J = 24.2, 5.7, 1.9 Hz, 1H), 3.46 (ddd, J = 24.2, 5.5, 1.7 Hz, 1H), 5.90 (q, J = 5.2 Hz, 1H).
$^{13}$C-NMR (50 MHz, $CDCl_3$): δ (ppm) = 10.9, 14.1 (2C), 20.7, 22.6, 23.0, 23.6, 24.9, 29.0, 29.1 (2C), 29.2, 30.3, 31.8, 34.6, 39.4, 71.7, 96.4, 173.5.

**Beispiel 4:**

Heptansäure-1-[(2-ethylhexyl)oxy]ethylester

$^1$H-NMR (200 MHz, $CDCl_3$): δ (ppm) = 0.81-0.94 (m, 9H), 1.22-1.39 (m, 14H), 1.39 (d, J = 5.3 Hz, 3H), 1.55-1.72 (m, 3H), 2.31 (t, J = 7.4 Hz, 2H), 3.33 (ddd, J = 28.5, 9.2, 3.2 Hz, 1H), 3.46 (ddd, J = 28.5, 9.2, 3.5 Hz, 1H), 5.90 (q, J = 5.2 Hz, 1H).
$^{13}$C-NMR (50 MHz, $CDCl_3$): δ (ppm) = 11.0, 14.0, 14.1, 20.7, 22.5, 23.0, 23.6, 24.9, 28.8, 29.0, 30.3, 31.5, 34.6, 39.4,

71.7, 96.3, 173.4.

**Beispiel 5:**

Essigsäure-1-(2-phenylethoxy)-heptylester

$^1$H-NMR (200 MHz, $CDCl_3$): δ (ppm) = 0.87 (t, J = 6.5 Hz, 3H), 1.22-1.34 (m, 8H), 1.58-1.72 (m, 2H), 2.05 (s, 3H), 2.90 (t, J = 7.2 Hz, 2H), 3.68 (ddd, J = 9.6, 7.3, 6.7 Hz, 1H), 3.88 (ddd, J = 9.6, 7.7, 7.1 Hz, 1H), 5.81 (t, J = 5.5 Hz, 1H), 7.18-7.30 (m, 5H).
$^{13}$C-NMR (50 MHz, $CDCl_3$): δ (ppm) = 14.0, 21.2, 22.5, 23.8, 28.8, 31.6, 34.4, 36.0, 70.1, 98.6, 126.1, 128.2 (2C), 128.8 (2C), 138.2, 170.8.

**Beispiel 6:**

Propionsäure-1-(2-phenylethoxy)-heptylester

$^1$H-NMR (200 MHz, $CDCl_3$): δ (ppm) = 0.87 (t, J = 6.5 Hz, 3H), 1.13 (t, J = 7.6 Hz, 3H), 1.20-1.34 (m, 8H), 1.58-1.72 (m, 2H), 2.33 (q, J = 7.6 Hz, 2H), 2.88 (t, J = 7.2 Hz, 2H), 3.68 (ddd, J = 7.1, 7.5, 9.5 Hz, 1H), 3.87 (ddd, J = 6.6, 7.4, 9.5 Hz, 1H), 5.83 (t, J = 5.5 Hz, 1H), 7.15-7.34 (m, 5H).
$^{13}$C-NMR (50 MHz, $CDCl_3$): δ (ppm) = 9.0, 14.0, 22.5, 23.9, 27.7, 28.9, 31.6, 34.4, 36.1, 70.1, 98.5, 126.1, 128.2 (2C), 128.8 (2C), 138.3, 174.1.

Die erfindungsgemäßen Depotpräparate wurden in zahlreiche Verbraucherprodukte eingearbeitet und deren anwendungstechnischen Eigenschaften mit verschiedenen Methoden untersucht. Bei der Herstellung der Formulierungen für die Verbraucherprodukte wurden molare Äquivalente der Aldehyde/Ketone und Alkohole in Form der 1-Alkoxyester bzw. in Form der freien Aldehyde/Ketone und Alkohole eingesetzt, um eine Vergleichbarkeit zu gewährleisten.

**Methode 1** Lagerstabilität

Die Lagerstabilität eines Riechstoffes bzw. eines Depotpräparates wird durch die prozentuale Menge des nach Lagerung noch vorhandenen Stoffes definiert.

$$\frac{\text{Menge nach der Lagerung}}{\text{Menge vor der Lagerung}} * 100\% = \text{Lagerstabilität}[\%]$$

Zur Bestimmung und zum Vergleich der Lagerstabilität werden sowohl das Depotpräparat (DP) bestehend aus einem oder mehreren 1-Alkoxyestern und die korrespondierenden Aldehyde/Ketone und Alkohole (AL) in separate Muster ($M_{DP}$ und $M_{AL}$) der gleichen Formulierung eines Verbraucherproduktes wie z.B. Waschmittel, Shampoo oder Seife eingearbeitet. Anschließend werden die separaten Muster in Portionen geteilt. Die eine Portion der Muster $M_{DP}$ und $M_{AL}$ werden unverzüglich einer geeigneten Extraktion und einer analytischen Messung unterzogen, um die Menge an Depotpräparat bzw. Aldehyd/Keton und Alkohol vor der Lagerung zu bestimmen. Bei der analytischen Untersuchung durch z.B. Gaschromatographie wird zur Quantifizierung ein geeigneter Standard verwendet. Die zweite Portion wird einer Lagerung bei erhöhter Temperatur für eine definierte Zeit unterzogen und anschließend mit den gleichen Methoden extrahiert und quantifiziert.

**Methode 2** Geruchliche Bewertung

Die geruchliche Bewertung eines Depotpräparates an sich bzw. im Vergleich mit den korrespondierenden Aldehyden/Ketonen und Alkoholen wird durch ein Gruppe geschulter Personen durchgeführt. Hierbei werden die geruchliche Stärke des Verbraucherproduktes bzgl. des verwendeten Aldehydes/Ketons und Alkohols in der Anwendung beurteilt. Das Verbraucherprodukt wird gemäß seiner Bestimmung zum Auftragen auf die Haut oder zum Waschen von Wäsche, Haut oder Haar verwendet. Während der Anwendung wird dann z.B. das Verbraucherprodukt selbst, dessen wässrige Lösungen, die feuchte oder trockene Wäsche bzw. die feuchte oder trockene Haut geruchlich auf einer Skala von 1 (schwacher Geruch) bis 6 (starker Geruch) beurteilt.

**Methode 3** Freisetzungsrate im Gasraum

Die analytische Messung der Konzentration von Riechstoffen selbst, den Depotpräparaten und den Riechstoffen freigesetzt aus den Depotpräparaten wird mittels der Gaschromatographie durchgeführt. Hierbei können weiterhin verschiedene Injektionsmethoden, wie z.B. die Thermodesorption, die Flüssiginjektion und die Gasinjektion verwendet werden.

Vor der analytischen Messung von Riechstoffen können verschiedene Anreicherungsverfahren wie z.B. die Extraktion, die Aufkonzentration oder die Adsorption verwendet werden. Als Extraktionsmittel für flüssig-flüssig oder flüssig-fest Extraktionen sind z.B. Lösungsmittel wie z.B. Kohlendioxid, Ether, Ketone, Kohlenwasserstoffe, Alkohole, Wasser und Ester geeignet.

Weiterhin kann durch Ausfrieren eines statischen oder dynamischen Gasraumes über dem parfümierten Produkt bzw. mit dem parfümierten Produkt behandelte Substrate wie Haar, Textilien oder Haut mittels Kühlfallen eine Anreicherung bzw. Aufkonzentrierung erreicht werden.

Für die Adsorption bzw. Extraktion von Riechstoffen aus einem statischen oder dynamischen Gasraum sind oberflächenaktive Adsorptionsmittel wie z.B. Haar, Textilien, Keramik, Kunststoffe, Poly-2,6-diphenyl-p-phenylenoxid (Tenax®), quervernetzte poröse Polymere auf Basis von Styrol, Ethylvinylbenzol, Vinylpyrrolidon, Vinylpyridin und Ethylenglykol-dimethacrylat (Poropak®-Serie) und Aktivkohle geeignet. Die an diesen Adsorptionsmitteln angereicherten Riechstoffe werden anschließend durch Wärme (Thermodesorption) oder Lösungsmittel desorbiert und können dann analysiert werden.

**Beispiel 7: Pulverwaschmittel**

Die Waschmittel A und B wurden sowohl direkt als auch nach Lagerung für vier Wochen zum Waschen verwendet bzw. analytisch untersucht.

Tabelle 1:

| Pulverwaschmittelformulierung | | | |
|---|---|---|---|
| **Inhaltsstoffe** | | **A** | **B** |
| Pulverwaschmittel | | 99,7 | 99,78 |
| DP Beispiel 5 (4) | Essigsäure-1-(2-phenylethoxy)-heptylester | 0,3 | |
| Aldehyd C7 (4) | n-Heptanal | | 0,12 |
| Phenylethylalkohol (4) | 2-Phenylethanol | | 0,13 |

**Geruchliche Beurteilung:**

Baumwollgewebe und synthetisches Gewebe wurden zusammen in zwei verschiedenen Maschinen zeitgleich mit den oben genannten Waschmitteln A und B gewaschen und im feuchten und im trockenen Zustand geruchlich bewertet: Die geruchliche Stärke der Wäschestücke, die mit dem Waschmittel A mit Depotpräparat gewaschen wurden war deutlich höher als die geruchliche Stärke der Wäschestücke, die mit dem Waschmittel B mit dem freien Aldehyd und freien Alkohol gewaschen wurden.

**Lagerstabilität:**

Die Lagerstabilität des freien Aldehydes nach einem Monat betrug 27%. Die aus dem freien Aldehyd gebildete Säure erzeugte eine unangenehme geruchliche Note. Die Lagerstabilität des freien Alkohols betrug 91%. Die Lagerstabilität des Aldehydes und des Alkohols im Depotpräparat betrug 93% und war damit deutlich höher.

**Freisetzungsrate:**

Die gewaschenen Textilien A und B wurden jeweils in separate Glasflaschen überführt. Anschließend wurde mittels SPME-Analyse ("Solid Phase Microextraction") bzw. direkter Gasraumanalyse die relative Konzentration an freiem Aldehyd und freiem Alkohol analysiert. In dem Gasraum über der mit dem Waschmittel A gewaschenen feuchten Wäsche wurden über fünfmal mehr freier Aldehyd und freier Alkohol gefunden. In dem Gasraum über der mit dem Waschmittel A gewaschenen trockenen Wäsche wurden siebenmal mehr des Aldehydes und des Alkohols gefunden.

**Beispiel 8: Seife**

Die folgende Seifenformulierung kann nach allgemein bekannten Methoden hergestellt werden. Die Angaben beziehen sich auf Gewichtsprozente. Die erhaltenen Seifen A und B wurden sowohl direkt als auch nach Lagerung für

vier Wochen zum Waschen verwendet bzw. analytisch untersucht.

Tabelle 2:

| Seifenformulierung | | | |
|---|---|---|---|
| **Inhaltsstoffe** | | **A** | **B** |
| Seifenbase (1) | Sodium Tallowate | 75,0 | 75,0 |
| Seifenbase (1) | Sodium Cocoate | 10,0 | 10,0 |
| Wasser | Water | 13,0 | 13,0 |
| Bayertitan AZ (2) | Titanium Dioxide | 0,3 | 0,3 |
| Tinopal CBS-X (3) | Disodium Distyrylbiphenyl Disulfonate | 0,2 | 0,2 |
| DP Beispiel 5 (4) | Essigsäure-1-(2-phenylethoxy)-heptylester | 0,3 | |
| Aldehyd C7 (4) | n-Heptanal | | 0,12 |
| Phenylethylalkohol (4) | 2-Phenylethanol | | 0,13 |

**Lieferanten:**

(1) Enzian Seifenfabrik, 72555 Metzingen, Germany
(2) Bayer AG, Bayerwerk, D-51368 Leverkusen, Germany
(3) Ciba Spezialitätenchemie AG, 4000 Basel, Switzerland
(4) Haarmann & Reimer GmbH, D-37603 Holzminden, Germany
(5) Quest International, Ashford, England

**Geruchliche und farbliche Beurteilung:**

Die Seifenformulierungen A und B wurden für ca. drei Monate bei Raumtemperatur gelagert.

Die Seife A welche das Depotpräparat beinhaltet zeigte farblich nicht oder nur geringfügige Veränderung während die Seife B eine gelbliche bzw. graue Verfärbung hatte. Durch die Verwendung des Depotpräparates wird eine Farbstabilität erzielt.

Nach der Lagerung wurden jeweils 1g der Seifen in 100g handwarmen Wasser aufgelöst bzw. die Seifenstücke zum Waschen von Haut verwendet.

In allen Fällen war der Dufteindruck über der wässrigen Lösungen der Seife A, welche das Depotpräparat beinhaltet, deutlich stärker als der Dufteindruck der Seife B, welche den freien Aldehyd und Alkohol beinhaltet.

Der Dufteindruck von der gewaschenen Haut, welche mit der Seife A gewaschen wurde, war ebenfalls höher und lang anhaltender als der Dufteindruck nach Waschen mit der Seife B.

**Lagerstabilität:**

Die Seifenformulierungen A und B wurden für ca. einen Monat bei Raumtemperatur in der Dunkelheit gelagert. Das Depotpräparat zeigte eine deutlich höhere Lagerstabilität als der korrespondierende Aldehyd.

Tabelle 3:

| Lagerstabilität von Depotpräparat und freiem Aldehyd in Seife | | |
|---|---|---|
| **Inhaltsstoffe** | | **Lagerstabilität [%]** |
| DP Beispiel 5 (4) | Essigsäure-1-(2-phenylethoxy)-heptylester | 93 |
| Aldehyd C7 (4) | n-Heptanal | 27 |
| Phenylethylalkohol (4) | 2-Phenylethanol | 91 |

**Freisetzungsrate:**

Zur Bestimmung der Hydrolysegeschwindigkeit wurde das Depotpräparat zu einer 1 %igen wässrigen Seifenlösung gegeben und die Konzentration des Depotpräparates bzw. des freien Aldehydes durch SPME-Analyse im

Gasraum gemessen.

Das Depotpräparat in der Seife A zeigte eine spontane Hydrolyse zu dem korrespondierenden Aldehyd und Alkohol. Bereits nach 5 Minuten war das Depotpräparat vollständig hydrolysiert.

Durch geeignete Wahl der Reste im Depotpräparat kann auch eine verzögerte bzw. unvollständige Hydrolyse erzielt werden. Hierdurch kann dann im Waschprozess ein Teil des Depotpräparates auf die Haut übertragen werden und anschließend durch die langsamere Spaltung der 1-Alkoxyester ein dauerhafter Dufteindruck auf der Haut erzielt werden.

**Beispiel 9: Shampoo**

Die folgende Shampooformulierung kann nach allgemein bekannten Methoden hergestellt werden. Die Angaben beziehen sich auf Gewichtsprozente.

Tabelle 4:

| Shampooformulierung | | | |
|---|---|---|---|
| **Inhaltsstoffe** | | **A** | **B** |
| Plantacare PS 10 (1) | Sodium Laureth Sulfate (and) Lauryl Glycoside | 20 000 | 20 000 |
| Entmineralisiertes Wasser | Water (Aqua) | bis 100 % | bis 100 % |
| Natriumchlorid | Sodium Chloride | 1,400 | 1,400 |
| Citronensäure 10,0 % Lösung | Citric Acid | 1,650 | 1,650 |
| Phenonip (2) | Phenoxyethanol (and) Methyl paraben (and) Ethylparaben (and) Propylparaben (and) Butylparaben | 0,500 | 0,500 |
| DP Beispiel 5 (4) | Essigsäure-1-(2-phenylethoxy)-heptylester | 0,3 | |
| Aldehyd C7 (4) | n-Heptanal | | 0,12 |
| Phenylethylalkohol (4) | 2-Phenylethanol | | 0,13 |

**Geruchliche Beurteilung:**

Die erhaltenen Shampoos A und B wurden zum Waschen von Haarsträhnen bzw. in einem sogenannten Halbseitenwaschtest an Probanden verwendet. Bei dem Halbseitenwaschtest wurde den Probanden jeweils eine Hälfte der Haare mit Shampoo A und die andere Hälfte der Haare mit Shampoo B gewaschen.

Die mit Shampoo B gewaschenen Haarsträhnen hatten direkt nach der Wäsche eine geringfügig höhere geruchliche Stärke im Vergleich zu den Haarsträhnen welche mit dem Shampoo A gewaschen wurden. Allerdings nahm die geruchliche Intensität der mit Shampoo B gewaschenen Haare rasch ab und war nach ca. vier Stunden nicht mehr von einer neutralen Probe unterscheidbar.

Die gleiche Duftbewertung ergab sich auch für den Halbseitenwaschtest an Probanden. Allerdings wurde von der Hälfte, die mit dem Shampoo A gewaschen wurde ein lang andauernder Aldehyd- wie auch Alkoholgeruch wahrgenommen. Auch noch nach mehreren Tagen war ein deutlicher Geruch des Aldehydes und des Alkohols auf der Haarhälfte A wahrnehmbar. Durch die höhere Übertragung und anschließende langsame Spaltung des Depotpräparates zum freien Aldehyd kann ein lang anhaltender Dufteindruck erzielt werden. Hierdurch zeigt sich der Vorteil des Depotpräparates gegenüber dem freien Aldehyd und freien Alkohol in der Anwendung.

**Patentansprüche**

**1.** Depotpräparate enthaltend mindestens eine Verbindung der Formel (I)

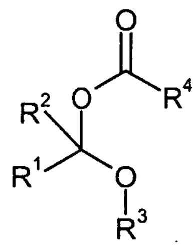

(I)

worin

$R^1$, $R^3$ und $R^4$ - unabhängig voneinander - einen organischen Rest mit 1 bis 30 Kohlenstoffatomen bedeuten,

$R^2$ Wasserstoff oder einen organischen Rest mit 1 bis 30 Kohlenstoffatomen bedeutet

und die Verbindung der Formel (I) nach Hydrolyse oder enzymatischer Spaltung neben dem Aldehyd oder Keton noch einen Alkohol und eine Carbonsäure freisetzt.

2. Depotpräparate nach Anspruch 1, **dadurch gekennzeichnet, dass** in der Formel (I)

$R^1$ und $R^3$ unabhängig voneinander für einen gesättigten oder ungesättigten, linearen oder verzweigten, alicyclischen oder aromatischen Rest mit 1 bis 18 Kohlenstoffatomen stehen,

$R^2$ für Wasserstoff, einen gesättigten oder ungesättigten, linearen oder verzweigten, alicyclischen oder aromatischen Rest mit 1 bis 18 Kohlenstoffatomen steht,

$R^4$ für einen gesättigten oder ungesättigten, linearen oder verzweigten, alicyclischen oder aromatischen Rest mit 1 bis 22 Kohlenstoffatomen steht.

3. Depotpräparate nach Anspruch 1, **dadurch gekennzeichnet, dass** die Reste $R^1$ und $R^2$ einen Aldehyd $R^1COH$ oder ein Keton $R^1R^2CO$ freisetzen, die ein Molekulargewicht von 100 g/mol bis 350 g/mol haben.

4. Depotpräparate nach Anspruch 3, **dadurch gekennzeichnet, dass** die Reste $R^1$ und $R^2$ einen Aldehyd $R^1COH$ oder ein Keton $R^1R^2CO$ freisetzen, die ein Molekulargewicht von 120 g/mol bis 270 g/mol haben.

5. Riechstoffe oder Parfümöle, **dadurch gekennzeichnet, dass** diese neben einem Depotpräparat der Ansprüche 1 bis 4 Extrakte aus natürlichen Rohstoffen, Kohlenwasserstoffe, aliphatische Alkohole, aliphatische Ketone, aliphatische Nitrile, aliphatische Carbonsäuren, acyclische Terpenalkohole, acyclische Terpenaldehyde, cyclische Terpenaldehyde, cyclische Alkohole, cycloaliphatische Alkohole, cyclische oder cycloaliphatische Ether, cyclische Ketone, cycloaliphatische Aldehyde oder Ketone, Ester cyclischer Alkohole oder Carbonsäuren, aromatische Kohlenwasserstoffe, araliphatische Alkohole, Ester araliphatischer Alkohole mit aliphatischen Carbonsäuren, aromatische oder araliphatische Aldehyde, Ketone oder Carbonsäuren, stickstoffhaltige, aromatische Verbindungen, Phenole, Phenylether oder Phenylester, heterocyclische Verbindungen oder Lactone enthalten.

6. Riechstoffe oder Parfümöle nach Anspruch 5, **dadurch gekennzeichnet, dass** diese in flüssiger Form unverdünnt oder mit einem Lösungsmittel verdünnt eingesetzt werden.

7. Riechstoffe oder Parfümöle nach Anspruch 5, **dadurch gekennzeichnet, dass** diese an einen Träger adsorbiert sind.

8. Riechstoffe oder Parfümöle nach Anspruch 5, **dadurch gekennzeichnet, dass** diese mikroverkapselt oder sprühgetrocknet sind oder als Einschluss-Komplex oder als Extrusions-Produkt vorliegen.

9. Verwendung der Riechstoffe oder Parfümöle nach einem der Ansprüche 5 bis 8 zur Herstellung von kosmetischen Pflegeprodukten, Haushaltsprodukten, Verpackungsprodukten oder Nahrungsmitteln.

10. Verfahren zur Herstellung von Verbindungen der Formel (I) aus Anspruch 1, **dadurch gekennzeichnet, dass** man

einen Aldehyd oder ein Keton mit 2 bis 4 Äquivalenten Alkohol in Gegenwart von katalytischen Mengen Säure zu dem entsprechenden Acetal oder Ketal umsetzt, unter saurer Katalyse aus dem Acetal oder Ketal ein Äquivalent Alkohol abspaltet und aus dem so entstandenen Enolether durch nachfolgende sauer katalysierte Umsetzung des Enolethers mit einer Carbonsäure zu den Verbindungen der Formel (I) gelangt.

**11.** Verfahren zur Herstellung von Verbindungen der Formel (I) aus Anspruch 1, **dadurch gekennzeichnet, dass** man durch Hydrid Reduktion eines Carbonsäureesters bzw. Umsetzung eines Carbonsäureesters mit einem metallorganischen Reagenz und anschließendem Abfang des sich bildenden Halbacetals mit einem Carbonsäureanhydrid zu den Verbindungen der Formel (I) gelangt.